# EUROPEAN PATENT SPECIFICATION

(11) **EP 2 089 032 B1**
(45) Date of publication and mention of the grant of the patent: **15.12.2010**
(21) Application number: 07766499.3
(22) Date of filing: 23.07.2007
(51) Int. Cl.: A61K 31/4545, A61K 31/19, A61K 31/40, A61K 31/519, A61K 31/551, A61K 31/554, A61P 25/08, A61P 25/18, A61P 25/24

(54) **A PHARMACEUTICAL COMPOSITION HAVING ANTIPSYCHOTIC, ANTIDEPRESSANT OR ANTIEPILEPTIC ACTIVITY WITH REDUCED SIDE EFFECT**
PHARMAZEUTISCHE ZUSAMMENSETZUNG MIT ANTIPSYCHOTISCHER, ANTIDEPRESSIVER ODER ANTIEPILEPTISCHER WIRKUNG MIT VERRINGERTER NEBENWIRKUNG
COMPOSITION PHARMACEUTIQUE DOTÉE D'UNE ACTIVITÉ ANTIPSYCHOTIQUE, ANTIDÉPRESSIVE OU ANTI-ÉPILEPTIQUE À EFFET SECONDAIRE RÉDUIT

(30) Priority: 02.11.2006 US 856177 P; 19.03.2007 US 687954
(43) Date of publication of application: 19.08.2009
(73) Proprietor: N-Gene Research Laboratories Inc., New York, NY 10022 (US)
(72) Inventor: LITERÁTI NAGY, Péter, H-1037 Budapest (HU); ROTH, Jesse, Whitestone, NY 11357 (US); SZILVASSY, Zoltán, H-4025 Debrecen-Józsa (HU); TORY, Kálmán, H-1137 Budapest (HU); BROWNSTEIN, Mike, Rockwille, MD 20852 (US); TAKACS, Kálmán, H-1082 Budapest (HU); VIGH, László, H-6726 Szeged (HU); MANDL, József, H-1132 Budapest (HU); SÜMEGI, Balázs, H-7634 Pécs (HU); BERNATH, Sándor, H-2089 Telki (HU); KOLONICS, Attila, H-1141 Budapest (HU); BALOGH, Gábor, H-6726 Szeged (HU); EGRI, János, H-1036 Budapest (HU)
(74) Representative: Beszédes, Stephan G.
(86) International application number: PCT/HU2007/000067
(87) International publication number: WO 2008/053257

(56) References cited:
- WO-A-03/007951

## Description

### Field of the invention

The invention refers to a pharmaceutical composition having antipsychotic, antidepressant or antiepileptic activity with reduced side effect and a use of O-(3-piperidino-2-hydroxy-1-propyl)-nicotinic amidoxime or a pharmaceutically acceptable acid addition salt thereof for the preparation of a pharmaceutical composition suitable for preventing or reducing the side effect leading to overweight or obesity in medication with a known antipsychotic, antidepressant or antiepileptic drug.

### Background of the invention

Antipsychotics are used for the treatment of psychiatric disorders, particularly schizophrenia, while antidepressants are administered to alleviate the symptoms of depression. Antiepileptic drugs prevent or reduce the severity and frequency of seizures in various types of epilepsy. Many patients treated with antipsychotics e.g. olanzapine or clozapine feel acoria due to a failure in the regulation of bod uptake, thus, the treatment frequently leads to weight gain. Overweight and even obesity may occur within 3-6 months after the beginning of the treatment as evidenced by reports on treated patients [Blin O.: A comparative review of new antipsychotics. Can. J. Psychiatry, 44, 235-44 (1999)]. In a similar manner, medication with many antidepressants e.g. amitriptyline, imipramine etc. or antiepileptics (anticonvulsants) e.g. valproic acid, sodium valproate etc. results in weight gain that may lead to obesity [Ruetsch O. et al., L'Encéphale, 31, 507-16 (2005)]. In case of adults, overweight is characterized by a body mass index of 25-30 kg/m², while a body mass index of above 30 kg/m² indicates obesity.

Overweight and obesity themselves are associated with hypertension and abnormal metabolic changes such as insulin resistance and dyslipidemia which are risk factors for diabetes. Obesity (particularly abdominal adiposis), insulin resistance and dyslipidemia are major features of "pre-diabetes" (metabolic syndrome) that leads to type 2 diabetes mellitus. Diabetes is associated with serious complications such as retinopathy, nephropathy, and neuropathy. In addition, diabetes is accompanied by increased mortality due to a greater risk of cardiovascular disease.

Due to the extensive and growing administration of antipsychotics, antidepressants and antiepileptic drugs to patients in the USA and throughout the developed countries, the above undesirable side effects are considered as an increasing problem which is related to increased rates of mortality and morbidity. In addition, patients requiring a treatment with an antipsychotic or an antidepressant or an antiepileptic drug may decide to stop treatment because of the induced weight gain.

Therefore, the aim of the invention is to provide a pharmaceutical composition having antipsychotic, antidepressant or antiepileptic activity with reduced side effect regarding weight gain that leads to overweight or obesity.

O-(3-piperidino-2-hydroxy-1-propyl)-nicotinic amidoxime) (abbreviated as BGP-15) was patented in 1976 as a new compound useful in the treatment of diabetic angiopathy, a complication of diabetes resulting in the damage of blood vessels. The basic patent is, among others, US-P No. 4,187,220.

US-P No. 6,306,878 refers to a method for the protection of the mitochondrial genome and/or mitochondrion from damage leading to myopathies and neurodegenerative diseases which comprises administering an effective non-toxic dose to a patient susceptible to such damage of an amidoximic acid derivative including BGP-15. A preferred myopathy is cardiomyopathy. Neurodegenerative diseases include Parkinson's disease, Huntington's disease and amyotrophic lateral sclerosis.

US-P No. 6,458,371 refers to a composition comprising 0.1-30 % of a hydroximic acid derivative including BGP-15 as the active ingredient and a carrier that is in the form of a cream, lotion, foam or spray. The composition is suitable for reducing the incidence of photodamage by radiation with UV-B.

US-P No. 6,884,424 refers to a method for preventing actinic keratosis by applying a hydroximic acid derivative including BGP-15 to the affected skin surface.

US-P No. 6,451,851 refers to a method of treating a patient suffering from a viral infection comprising administering to the patient a pharmaceutically effective amount of a known antivirally active agent together with a hydroximic acid derivative including BGP-15.

US-P No. 6,440,998 refers to a pharmaceutical composition having antitumor activity with reduced side effect comprising cisplatin or carboplatin and a hydroximic acid derivative including BGP-15. US-P No. 6,656,955 refers to a pharmaceutical composition having antitumor activity with reduced side effect comprising paclitaxel or docetaxel and a hydroximic acid derivative including BGP-15. US-P No. 6,720,337 refers to a pharmaceutical composition having antitumor activity with reduced side effect comprising oxaliplatin and a hydroximic acid derivative including BGP-15. US-P No. 6,838,469 refers to a pharmaceutical composition having antitumor activity with reduced side effect comprising pyrimidine derivatives and BGP-15.

PCT Patent Application published under No. WO 00107580 disclosed experimental data for the antidiabetic effect of BGP-15 in the treatment of type 1 diabetes mellitus. It is to be noted that type 1 diabetes mellitus is an autoimmune disease occuring at young age, while type 2 diabetes mellitus is a metabolic disease occuring at higher age.

PCT Application published under No. WO 03/007951 refers to a pharmaceutical combination of hydroximic acid derivatives including BGP-15 and an antidiabetic or anti-hyperlipidemic active agent for the prevention or treatment of a prediabetic state, metabolic X-syndrome or diabetes mellitus as well as disorders which are associated with the states listed above, namely endogenic metabolic disorders, insulin resistance, dislipidemia, alopecia, diffuse effluvium and/or female endocrine disorders based on androgenic preponderance. In the description, laboratory data indicate that BGP-15 enhances, synergistically, the effect of the known antidiabetic agent metformin and troglitazone, respectively. The laboratory data also show that BGP-15 in itself enhances the insulin sensitivity (thus, reduces the insulin resistance) in both normal and hyper-cholesterolemic animals relative to the control.

PCT Application published under No. WO 2005/122678 refers to the use of BGP-15 in a pharmaceutical composition having prokinetic effect (i.e. induces activity in the stomach and intestines). Prokinetic effect includes possible treatment of reflux esophagitis, gastroparesis, influencing bile flow from the gall bladder etc.

PCT Application published under No. WO 2005/123049 refers to the use of BGP-15 for mitochondrial genesis i.e. to increase the number of mitochondria in the cells resulting in a roborating effect.

PCT Application published under No. WO 2006/079910 refers to the use of BGP-15 for the treatment of lesions in the oral cavity, especially periodontal disease.

### Summary of the invention

It has been found that O-(3-piperidino-2-hydroxypropyl)-nicotinic amidoxime or a pharmaceutically acceptable acid addition salt thereof can be used for the prevention or reduction of weight gain or obesity in a patient treated with an antipsychotic drug or an antidepressant drug or an antiepileptic drug.

Thus, the invention provides the use of O-(3-piperidino-2-hydroxy-1-propyl)-nicotinic amidoxime or a pharmaceutically acceptable acid addition salt thereof for the preparation of a pharmaceutical composition suitable for preventing or reducing the side effect leading to overweight or obesity in medication with a known antipsychotic, antidepressant or antiepileptic drug.

Furthermore, the invention provides a pharmaceutical composition having antipsychotic or antidepressant or antiepileptic activity with reduced side effect comprising a known antipsychotic or antidepressant or antiepileptic drug and O-(3-piperidino-2-hydroxy-1-propyl)-nicotinic amidoxime or a pharmaceutically acceptable acid addition salt thereof in admixture with one or more conventional carrier(s).

### Description of preferred embodiments

An "antipsychotic" or "antipsychotic agent" refers to a drug used to treat severe mental disorders (psychoses) including schizophrenia and mania. Some antipsychotic agents are administered in small doses to relieve anxiety.

A preferred group of antipsychotics include phenothiazine derivatives of formula IA 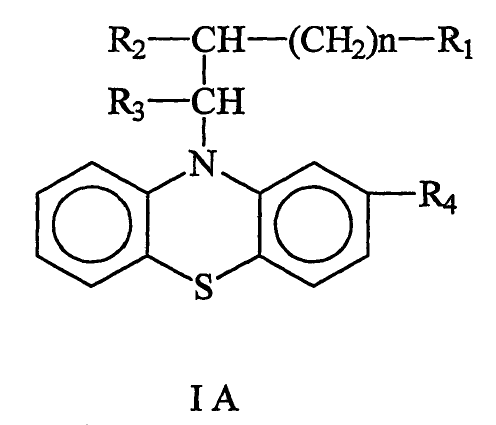 and pharmaceutically acceptable acid addition salts thereof, wherein
R₁ represents a di(C₁₋₄ alkyl)amino, 1-(C₁₋₄ alkyl)piperidyl, 4-(C₁₋₄ alkyl)piperazinyl or 4-[2-hydroxy(C₁₋₄ alkyl)]-1-piperazinyl group,
R₂ and R₃ stand, independently, for a hydrogen atom or C₁₋₄ alkyl group,
R₄ means a hydrogen or halo atom, carboxy, C₁₋₄ alkoxy, C₁₋₄ alkanoyl, trifluoromethyl, methylmercapto or methylsulfinyl group, and
n has a value of 0 or 1.

Suitably, in formula IA
R₁ represents a dimethylamino, 1-methylpiperidyl, 4-methylpiperazinyl or 4-(2-hydroxyethyl)-1-piperazinyl group, R₂ and R₃ stand, independently, for a hydrogen atom or methyl group,
R₄ means a hydrogen or chloro atom, carboxy, methoxy, acetyl, trifluoromethyl, methylmercapto or methylsulfinyl group, and
n has a value of 0 or 1.

Especially preferred phenothiazine derivatives are as follows:
chlorpromazine i.e. 2-chloro-N,N-dimethyl-10H-phenothiazine-10-propanamine,
promazine i.e. N,N-dimethyl-10H-phenothiazine-10-propanamine,
mesoridazine i.e. 10-[2-(1-methyl-2-piperidinyl)ethyl]-2-(methyl-sulfinyl)-10H-phenothiazine,
fluphenazine i.e. 4-[3-[2-(trifluoromethyl)-10H-phenothiazin-10-yl]propyl]-1-piperazineethanol, and
trifluoperazine i.e. 10-[3-(4-methyl-1-piperazinyl)propyl]-2-(trifluoromethyl)-10H-phenothiazine,
as well as pharmaceutically acceptable acid addition salts thereof.

Another preferred group of antipsychotics include thioxanthene derivatives of formula IB 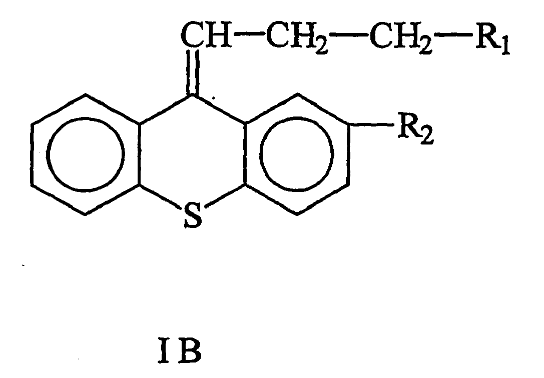 and pharmaceutically acceptable acid addition salts thereof, wherein
R₁ represent a di(C₁₋₄ alkyl)amino, 4-(C₁₋₄ alkyl)-1-piperazinyl, 4-[2-hydroxy(C₁₋₄ alkyl)]-1-piperazinyl, 4-[2-(C₁₋₄ alkanoyloxy)-(C₁₋₄ alkyl)]-1-piperazinyl or 4-(2-decanoyioxyethyl)-1-piperazinyl group,
R₂ stands for a halo atom, trifluoromethyl or N,N-dimethyl-sulfonylamido group.

Suitably, in formula IB
R₁ represent a dimethylamino, 4-methyl-1-piperazinyl, 4-(2-hydroxyethyl)-1-piperazinyl, 4-(2-acetoxyethyl)-1-piperazinyl or 4-(2-decanoyloxyethyl)-1-piperazinyl group,
R₂ stands for a chloro atom, trifluoromethyl or N,N-dimethylsulfonylamido group.

Especially preferred thioxanthene derivatives are as follows:
chlorprothixene i.e. 3-(2-chloro-9H-thioxanthen-9-ylidene)-N,N-dimethyl-1-propanamine,
clopenthixol i.e. 4-[3-(2-chloro-9H-thioxanthen-9-ylidene)-propyl]-1-piperazine-ethanol,
thiothixene i.e. N,N-dimethyl-9-[3-(4-methyl-1-piperazinyl)-propylidene]-thioxanthene-2-sulfonamide, and flupentixol i.e. 4-[3-(2-(trifluoromethyl)-9H-thioxanthen-9-ylidene)propyl]-1-piperazine-ethanol,
as well as pharmaceutically acceptable acid addition salts thereof.

A further preferred group of antipsychotics include compounds of formula IC 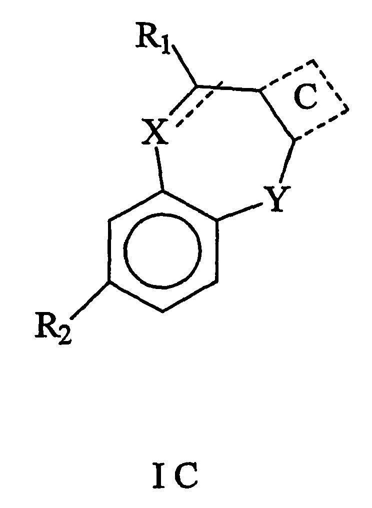 wherein
X stands for a nitrogen atom or a group of formula -C= or -CH-,
Y represents a group of formula -NH-, oxygen or nitrogen atom,
R₁ means a 4-(2-hydroxyethoxyethyl)-1-piperazinyl, 4-(C₁₋₄ alkyl)-1-piperazinyl or 4-(3-hydroxypropyl)-1-piperazinyl group,
R₂ is a hydrogen or halo atom,
ring C represents a benzene ring optionally substituted by a halo atom or N,N-dimethylsulfonamido group or ring C stands for a heterocyclic group that forms with the benzodiazepine portion a thieno[2,3-b][1,5]benzodiazepine structure, wherein the 5-membered thieno ring is optionally substituted in position 2 by a methyl group,
the dotted line between X and the adjacent carbon atom has no meaning in case of the saturated ring, otherwise the dotted line represents a valence bond,
and, if chemically possible, pharmaceutically acceptable acid addition salts thereof.

Suitably, in formula IC
R₁ means a 4-(2-hydroxyethoxyethyl)-1-piperazinyl, 4-(methyl)-1-piperazinyl or 4-(3-hydroxypropyl)-1-piperazinyl group,
R₂ is a hydrogen or chloro atom,
X, Y, ring C and the dotted line are as defined above.

Especially preferred derivatives of formula IC are as follows:
clozapine i.e. 8-chloro-11-(4-methyl-1-piperazinyl)-5H-dibenzo-[b,e][1,4]diazepine,
olanzapine i.e. 2-methyl-4-(4-methyl-1-piperazinyl)-10H-thieno-[2,3-b][1,5]-benzodiazepine,
quetiapine i.e. 2-[2-(4-dibenzo[b,f][1,4]thiazepin-11-yl-1-piperazinyl)ethoxy]-ethanol,
zotepine i.e. 2-[(.8-chlorodibenzo[b,f]thiepin-10-yl)oxy]-N,N-dimethylethanamine,
isoclozapine i.e. chloro-11-(4-methyl-1-piperazinyl)-5H-dibenzo[b,e][1,4]-diazepine,
clothiapine i.e. 2-chloro-11-(4-methyl-1-piperazinyl)dibenzo-[b,f][1,4]thiazepine,
oxithepine i.e. 10-[4-(3-hydroxypropyl)piperazino]-10,11-dihydrodibenzo[b,f]-thiepine,
and, if chemically possible, pharmaceutically acceptable acid addition salts thereof.

A still further preferred group of antipsychotics include benzamide derivatives of formula ID 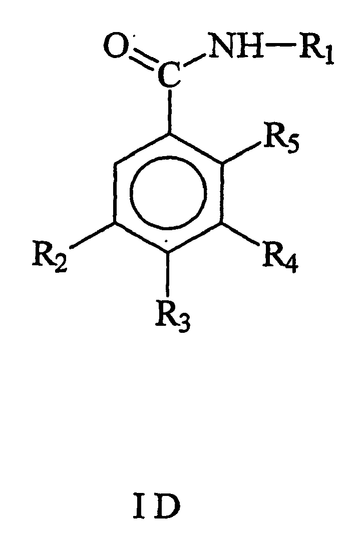 and pharmaceutically acceptable acid addition salts thereof, wherein
R₁ represents an N-[1-(C₁₋₄ alkyl)-2-pyrrolidinyl]-(C₁₋₄ alkyl), 2-[di(C₁₋₄ alkyl)-amino]-(C₁₋₄ alkyl) or 1-benzyl-3-pyrrolidinyl group,
R₂ stands for a hydrogen or halo atom, aminosulfonyl or (C₁₋₄ alkyl)sulfonyl group,
R₃ means a hydrogen or halo atom, amino or (C₁₋₄ alkyl)amino group,
R₄ is hydrogen or halo atom or methoxy group,
R₅ represents a C₁₋₄ alkoxy or allyloxy group.

Suitably, in formula ID
R₁ represents an N-(1-ethyl-2-pyrrolidinyl)methyl, 2-(diethylamino)ethyl or 1-benzyl-3-pyrrolidinyl group,
R₂ stands for a hydrogen or chloro atom, aminosulfonyl or ethylsulfonyl group,
R₃ means a hydrogen or chloro atom, amino or methylamino group,
R₄ is hydrogen or bromo atom or methoxy group,
R₅ represents a methoxy or allyloxy group.

Especially preferred derivatives of formula ID are as follows:
sulpiride i.e. 5-(aminosulfonyl)-N-[(1-ethyl-2-pyrrolidinyl)-methyl]-2-methoxy-benzamide,
amisulpride i.e. 4-amino-N-[(1-ethyl-2-pyrrolidinyl)methyl]-5-(ethylsulfonyl)-2-methoxybenzamide and
remoxipride i.e. (S)-3-bromo-N-[(1-ethyl-2-pyrrolidinyl)-methyl]-2,6-dimethoxybenzamide,
as well as pharmaceutically acceptable acid addition salts thereof.

A still further preferred group of antipsychotics consists of the benzisoxazole derivatives of formula IF 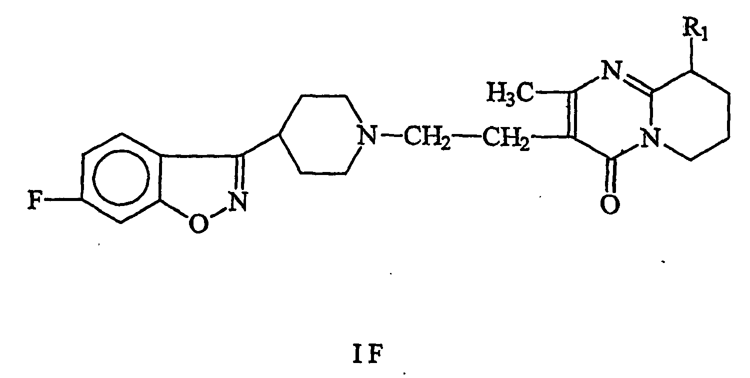 wherein
R₁ represents a hydrogen atom or hydroxy group.

Especially preferred benzisoxazole derivatives of formula IF are as follows:
risperidone i.e. 3-[2-[4-(6-fluoro-1,2-benzisoxazol-3-yl)-1-piperidinyl]-ethyl]-6,7,8,9-tetrahydro-2-methyl-4H-pyrido[1,2-a]-pyrimidin-4-one and
paliperidone i.e. 3-[2-[4-(6-fluoro-1,2-benzisoxazol-3-yl)-1-piperidinylethyl]-6,7,8,9-tetrahydro-9-hydroxy-2-methyl-4H-pyrido[1,2-a]pyrimidin-4-one.

A still further preferred group of antipsychotics consists of the diphenylbutyl-piperidine derivatives of formula IG 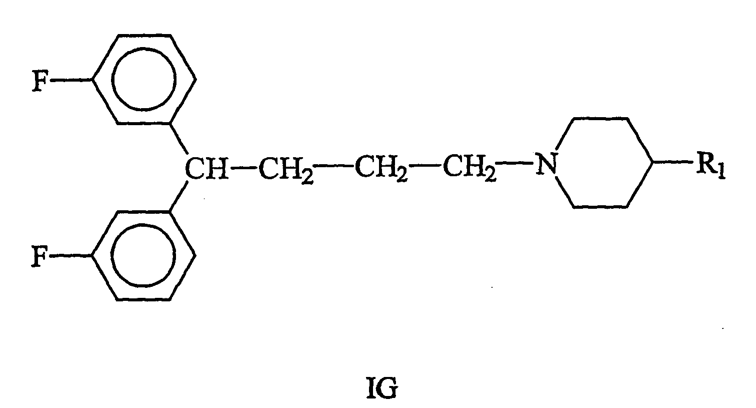 Wherein
R₁ represents a 2-benzimidazolon-1-yl group.

An especially preferred compound of formula IG is pimozide i.e. 1-[1-[4,4-bis(4-fluorophenyl)butyl]-4-piperidinyl]-1,3-dihydro-2H-benzimidazol-2-one.

A still further preferred group of antipsychotics include butyrophenone derivatives and pharmaceutically acceptable acid addition salts thereof such as the following compounds:
haloperidol i.e. 4-[4-(4-chlorophenyl)-4-hydroxy-1-piperidinyl]-1-(4-fluorophenyl)-1-butanone,
bromperidol i.e. 4-[4-(4-bromophenyl)-4-hydroxy-1-piperidinyl]-1-(4-fluorophenyl)-1-butanone or
trifluperidol i.e. 1-(4-fluorophenyl)-4-[4-hydroxy-4-[3-(trifluoromethyl)phenyl]-1-piperidinyl]-1-butanone.

A still further preferred group of antipsychotics include indole derivatives and pharmaceutically acceptable acid addition salts thereof such as the following compounds:
molindone e.g. 3-ethyl-1,5,6,7-tetrahydro-2-methyl-5-(4-morpholinylmethyl)-4H-indol-4-one,
ziprasidone e.g. 5-[2-[4-(1,2-benzisothiazol-3-yl)-1-piperazinyl]-ethyl]-6-chloro-1,3-dihydro-2H-indol-2-one,
sertindole e.g. 1-[2-[4-[5-chloro-1-(4-fluorophenyl)-1H-indol-3-yl]-1-piperidinyl]-ethyl]-2-imidazolidinone or
oxypertine i.e. 5,6-dimethoxy-2-methyl-3-[2-(4-phenyl-1-piperazinyl)ethyl]-1H-indole.

The term "antidepressant" or "antidepressant agent" refers to a drug that alleviates the symptoms of depression. A preferred group of antidepressants includes bicyclic compounds such as
paroxetine i.e. (3S-trans)-3-[(1,3-benzodioxol-5-yloxy)methyl]-4-(4-fluorophenyl)-piperidine,
citalopram i.e. 1-[3-(dimethylamino)propyl]-1-(4-fluorophenyl)-1,3-dihydro-5-isobenzofurancarbonitrile,
sertraline i.e. (1S-cis)-4-(3,4-dichlorophenyl)-1,2,3,4-tetrahydro-N-methyl-1-naphthalenamine
and pharmaceutically acceptable acid addition salts thereof.

Another preferred group of antidepressants include tricyclic compounds e.g.
amitriptyline i.e. 3-(10,11-dihydro-5H-dibenzo[a,d]cyclohepten-5-ylidene)-N,N-dimethyl-1-propanamine,
doxepin i.e. 3-dibenz[b,e]oxepin-11(6H)ylidene-N,N-dimethyl-propanamine,
imipramine i.e. 10,11-dihydro-N,N-dimethyl-5H-dibenz[b,f]-azepine-5-propan-amine,
clomipramine i.e. 3-chloro-10,11-dihydro-N,N-dimethyl-5H-dibenz[b,f]azepine-5-propanamine,
nortriptyline i.e. 3-(10,11-dihydro-5H-dibenzo[a,d]cyclohepten-5-ylidene)-N-methyl-1-propanamine,
trimipramine i.e. 10,11-dihydro-N,N,β-trimethyl-5H-dibenz[b,f]-azepine-5-propanamine, or
desipramine i.e. 10,11-dihydro-N-methyl-5H-dibenz[b,f]-azepine-5-propanamine,
as well as pharmaceutically acceptable acid addition salts thereof.

A further preferred group of antidepressants includes tetracyclic compounds such as
maprotiline e.g. N-methyl-9,10-ethanoanthracene-9(10H)-propanamine
and pharmaceutically acceptable acid addition salts thereof.

Further preferred antidepressants include e.g.
fluoxetine i.e. (±)-N-methyl-γ-[4-(trifluoromethyl)phenoxy]-benzenepropanamine,
fluvoxamine i.e. (E)-5-methoxy-1-[4-(trifluoromethyl)phenoxy]-1-pentanone O-(2-aminoethyl)oxime
and pharmaceutically acceptable acid addition salts thereof.

"The term "antiepileptic" or "anticonvulsant" or "antiepileptic agent" refers to a drug that prevents or reduces the severity and frequency of seizures in various types of epilepsy. The term "antiepileptic" is preferred since not all seizures involve convulsions. A preferred group of antiepileptics includes certain phenothiazine derivatives of formula IA such as triflupromazine i.e. N,N-dimethyl-2-(trifluoromethyl)-10H-phenothiazine-10-propan-amine and metofenazate i.e. 3,4,5-trimethoxybenzoic acid 2-[4-[3-(2-chloro-10H-phenothiazin-10-yl)propyl]-1-piperazinyl]ethyl ester which latter is typically administered as the difumarate. Said phenothiazine derivatives possess, in addition to antipsychotic, also antiepileptic activity.

A further preferred group of antiepileptics includes benzodiazepine derivatives such as clonazepam i.e.5-(2-chloro-phenyl)-1,3-dihydro-7-nitro-2H-1,4-benzodiazepine-2-one, clobazam i.e. 7-chloro-1-methyl-5-phenyl-1H-1,5-benzodiazepine-2,4(3H,5H)-dione etc., dibenzazepine derivatives such as carbamazepine i.e. 5H-dibenz[b,f]azepine-5-carboxamide having also analgesic activity, oxcarbazepine i.e. 10,11-dihydro-10-oxo-5H-dibenz[b,f]azepine-5-carboxamide etc., barbituric acid derivatives having also hypnotic and sedative activity such as phenobarbital i.e. 5-ethyl-5-phenyl-2,4,6(1H,3H,5H)-pyrimidine-trione and pharmaceutically acceptable metal salts thereof, eterobarb i.e. 5-ethyl-1,3-bis(methoxy-methyl)-5-phenyl-2,4,6(1H,3H,5H)-pyrimidine-trione, proxibarbal i.e. 5-(2-hydroxy-propyl)-5-(2-propenyl)-2,4,6(1H,3H,5H)-pyrimidinetrione, primidone ie.5-ethyl-dihydro-5-phenyl-4,6(1H,5H)-pyrimidinedione etc., hydantoin derivatives such as phenytoin i.e. 5,5-diphenyl-2,4-imidazolidinedione, mephenytoin i.e.5-ethyl-3-methyl-5-phenyl-2,4-imidazolidinedione, fosphenytoin i.e. 5,5-diphenyl-3-phosphonoyl-methyl-2,4-imidazolidinedione etc. and pharmaceutically acceptable metal salts thereof, oxazolidine derivatives such as ethadione i.e. 3-ethyl-5,5-dimethyl-2,4-oxazolidinedione etc., succinimide derivatives such as ethosuximide i.e. 3-ethyl-3-methyl-2,5-pyrrolidine-dione, phensuximide i.e. 1-methyl-3-phenyl-2,5-pyrrolidinedione etc., carboxylic acid derivatives such as valproic acid i.e. 2-propylpentanoic acid and pharmaceutically acceptable metal salts thereof, valpromide i.e. 2-propylpentanamide, valnoctamide i.e. 2-ethyl-3-methyl-pentanamide etc.

An additional useful group of antiepileptics includes gamma-aminobutyric acid (GABA) derivatives such as gabapentin i.e. 1-(aminomethyl)cyclohexaneacetic acid, progabide i.e. 4-[[(4-chlorophenyl)(5-fluoro-2-hydroxyphenyl)-methylene]amino]butanamide, vigabatrin i.e. 4-amino-5-hexenoic acid, piracetam i.e. 2-oxo-1-pyrrolidineacetamide, oxiracetam i.e. 4-hydroxy-2-oxo-1-pyrrolidineacetamide, nefiracetam i.e. N-(2,6-dimethyl-phenyl)-2-oxo-1-pyrrolidine-acetamide etc. and pharmaceutically acceptable metal salts of the acids, carbamate derivatives such as meprobamate i.e. 2-methyl-2-propyl-1,3-propanediol dicarbamate having also anxiolytic effect, felbamate i.e. 2-phenyl-1,3-propanediol dicarbamate etc., some sulfonamides such as acetazolamide i.e. N-[5-(aminosulfonyl)-1,3,4-thiadiazol-2-yl]acetamide, zonisamide i.e. 1,2-benzisoxazole-3-methanesulfonamide), sulthiame i.e. 4-(tetrahydro-2H-1,2-thiazin-2-yl)-benzenesulfonamide S,S-dioxide etc., N-acylurea derivatives such as phenacemide i.e. N-(amino-carbonyl)benzene-acetamide), pheneturide i.e. N-(amino-carbonyl)-α-ethylbenzeneacetamide etc.

Additional useful antiepileptics include lamotrigine i.e. 6-(2,3-dichlorophenyl)-1,2,4-triazine-3,5-diamine, topiramate i.e. 2,3:4,5-bis-O-(1-methylethylidene)-β-D-fructopyranose sulfamate, and tiagabine i.e. (R)-1-[4,4-bis(3-methyl-2-thienyl)-3-butenyl]-3-piperidinecarboxylic acid and pharmaceutically acceptable metal salts thereof.

An especially preferred group of antiepileptics includes valproic acid and pharmaceutically acceptable alkali metal valproates."

A pharmaceutically acceptable acid addition salt is a salt formed with a pharmaceutically acceptable inorganic acid such as hydrochloric acid, sulfuric acid etc. or with a pharmaceutically acceptable organic acid such as acetic acid, lactic acid, tartaric acid etc. Preferred acid addition salts include hydrochlorides, acetates, maleates etc. A preferred acid addition salt of O-(3-piperidino-2-hydroxy-1-propyl)-nicotinic amidoxime is the dihydrochloride thereof.

A pharmaceutically acceptable metal salt is a salt formed with a pharmaceutically acceptable inorganic base. Preferred metal salts include alkali metal salts such as sodium or potassium salt.

If chemically possible, the active agents used according to the invention can be also in the form of the pharmaceutically acceptable acid addition salt or metal salt thereof.

BGP-15 can be prepared by the process described in e.g. US-P No. 4,187,220.

In one embodiment, a conventional dose of a known antipsychotic or antidepressant or antiepileptic agent is administered to a patient requiring treatment with an antipsychotic or antidepressant or antiepileptic drug, and, simultaneously, a dose of BGP-15 or a pharmaceutically acceptable acid addition salt thereof is administered. This non-toxic dose of BGP-15 prevents or reduces, effectively, the weight gain associated with the administration of the antipsychotic or antidepressant or antiepileptic drug leading otherwise to overweight or even obesity.

Generally, the daily dose of antipsychotic, antidepressant or antiepileptic agents for an adult person of about 70 kg body weight amounts to 1-1000 mg. The similar daily dose of BGP-15 (as dihydrochloride) is, in general, 5-1000 mg, preferably 50-500 mg.

According to certain embodiments, 10-20 mg of olanzapine or 100-800 mg of clozapine and 50-500 mg of BGP-15 dihydrochloride are administered to an adult, daily.

In a further embodiment, O-(3-piperidino-2-hydroxy-1-propyl)-nicotinic amidoxime or a pharmaceutically acceptable acid addition salt thereof is used for the preparation of a pharmaceutical composition suitable for preventing or reducing the side effect leading to overweight or obesity in medication with a known antipsychotic, antidepressant or antiepileptic drug. This pharmaceutical composition is administered to a patient treated with an antipsychotic, antidepressant or antiepileptic drug to prevent the side effect thereof leading to overweight or obesity. The daily dose of the pharmaceutical composition comprising BGP-15 or a pharmaceutically acceptable acid addition salt thereof is similar as given above.

A still further embodiment consists in a pharmaceutical composition having antipsychotic or antidepressant or antiepileptic activity with reduced side effect comprising a known antipsychotic or antidepressant or antiepileptic agent and O-(3-piperidino-2-hydroxy-1-propyl)-nicotinic amidoxime or a pharmaceutically acceptable acid addition salt thereof in admixture with one or more conventional carrie(s).

In case of the pharmaceutical composition having antipsychotic or antidepressant or antiepileptic activity with reduced side effect
either each of the two active agents (i.e. the known antipsychotic or antidepressant or antiepileptic drug and BGP-15) has been converted, one by one, to separate pharmaceutical compositions using one or more conventional carrier(s) and any of the usual processes of drug manufacture, and in this case the two sorts of pharmaceutical composition obtained are administered to the patient simultaneously or one after the other;
or the two active agents have been converted to one single pharmaceutical composition that can be administered to the patient being in need thereof. In the latter case, the pharmaceutical composition may contain a mixture of the two active agents, or each of the active agents may be present at a different site in the pharmaceutical composition, e.g. one of them in the tablet core and the other in a coating of the tablet core. Of course, one or more conventional carriers and any of the usual processes of drug manufacture are used to prepare this single pharmaceutical composition.

The pharmaceutical composition having antipsychotic or antidepressant or antiepileptic activity with reduced side effect contains an antipsychotic or antidepressant or antiepileptic drug or, if chemically possible, a pharmaceutically acceptable acid addition salt or metal salt thereof and BGP-15 or a pharmaceutically acceptable acid addition salt thereof in addition to one or more pharmaceutically acceptable carrier(s). The pharmaceutical composition may include any dosage form suitable for peroral, parenteral or rectal administration or for local treatment, and can be solid or liquid. The weight ratio of the known antipsychotic or antidepressant or antiepileptic agent and BGP-15 or a pharmaceutically acceptable acid addition salt thereof is, in general, (1-1000):(1000-1).

In principle, the pharmaceutical composition of the invention may contain more then one antipsychotic and/or antidepressant and/or antiepileptic drugs.

The solid pharmaceutical compositions suitable for peroral administration may be powders, capsules, tablets, film-coated tablets, microcapsules etc., and can comprise binding agents such as gelatine, sorbitol, poly(vinylpyrrolidone) etc.; filling agents such as lactose, glucose, starch, calcium phosphate etc.; auxiliary substances for tabletting such as magnesium stearate, talc, poly(ethylene glycol), silica etc.; wetting agents such as sodium laurylsulfate etc. as the carrier.

The liquid pharmaceutical compositions suitable for peroral administration may be solutions, suspensions or emulsions and can comprise e.g. suspending agents such as gelatine, carboxymethylcellulose etc.; emulsifiers such as sorbitane monooleate etc.; solvents such as water, oils, glycerol, propylene glycol, ethanol etc.; preservatives such as methyl p-hydroxybenzoate etc. as the carrier.

Pharmaceutical compositions suitable for parenteral administration consist of sterile solutions of the active ingredients, in general.

Dosage forms listed above as well as other dosage forms are known *per se,* see e.g. Remington's Pharmaceutical Sciences, 18th Edition, Mack Publishing Co., Easton, USA (1990).

The pharmaceutical composition contains dosage unit, in general. The daily dose can be administered in one or more portions. The actual dosage depends on many factors and is determined by the doctor.

The pharmaceutical composition is prepared by admixing the active ingredients to one or more carrier(s), and converting the mixture obtained to a pharmaceutical composition in a manner known *per se.* Useful methods are known from the literature, e.g. Remington's Pharmaceutical Sciences mentioned above.

A preferred pharmaceutical composition of the invention having antipsychotic activity with reduced side effect contains an antipsychotic agent selected from the group consisting of chlorpromazine, promazine, mesoridazine, fluphenazine, trifluoperazine, chlorprothixene, clopenthixol, thiothixene, flupentixol, clozapine, olanzapine, quetiapine, zotepine, isoclozapine, clothiapine, oxithepine, sulpiride, amisulpride, remoxipride, risperidone, paliperidone, pimozide, haloperidol, bromperidol, trifluperidol, molindone, ziprasidone, sertindole and oxipertine or a pharmaceutically acceptable acid addition salt thereof in addition to BGP-15 or a pharmaceutically acceptable acid addition salt thereof and one or more conventional carrier(s).

A still preferred pharmaceutical composition of the invention having antipsychotic activity with reduced side effect contains an antipsychotic agent selected from the group consisting of olanzapine, clozapine, risperidone, quetiapine and sulpiride or a pharmaceutically acceptable acid addition salt thereof in addition to BGP-15 or a pharmaceutically acceptable acid addition salt thereof and one or moreconventional carrier(s).

An especially preferred pharmaceutical composition of the invention having antipsychotic activity with reduced side effect contains an antipsychotic agent selected from the group consisting of olanzapine and clozapine in addition to BGP-15 or a pharmaceutically acceptable acid addition salt thereof and one or more conventional carrier(s).

A preferred pharmaceutical composition of the invention having antidepressant activity with reduced side effect contains an antidepressant agent selected from the group consisting of paroxetine, citalopram, fluoxetine, fluvoxamine, sertraline, amitriptyline, doxepine, imipramine, clomipramine, nortriptyline, trimipramine, desipramine and maprotiline or a pharmaceutically acceptable acid addiction salt thereof in addition to BGP-15 or a pharmaceutically acceptable acid addition salt thereof and one or more conventional carrier(s).

A still preferred pharmaceutical composition of the invention having antidepressant activity with reduced side effect contains an antidepressant agent selected from the group consisting of clomipramine, citalopram, fluoxetine, fluvoxamine, paroxetine and sertraline or a pharmaceutically acceptable acid addition salt thereof in addition to BGP-15 or a pharmaceutically acceptable acid addition salt thereof and one or more conventional carrier(s).

An especially preferred pharmaceutical composition of the invention having antidepressant activity with reduced side effect contains an antidepressant agent selected from the group consisting of clomipramine, citalopram, fluvoxamine and paroxetine or a pharmaceutically acceptable acid addition salt thereof in addition to BGP-15 or a pharmaceutically acceptable acid addition salt thereof and one or more conventional carrier(s).

A preferred pharmaceutical composition of the invention having antiepileptic activity with reduced side effect contains an antiepileptic agent selected from the group consisting of triflupromazine, metofenazate, clonazepam, clobazam, carbamazepine, oxcarbazepine, phenobarbital, eterobarb, proxibarbal, primidone, phenytoin, mephenytoin, fosphenytoin, ethadione, ethosuximide, phensuximide, valproic acid, valpromide, valnoctamide, gabapentin, progabide, pregabalin, vigabatrin, oxiracetam, nefiracetam, meprobamate, felbamate, acetazolamide, zonisamide, sulthiame, phenacemide, pheneturide, lamotrigine, topiramate and tiagabine in addition to BGP-15 or a pharmaceutically acceptable acid addition salt or metal salt thereof and one or more conventional carrier(s).

A more preferred pharmaceutical composition of the invention having antiepileptic activity with reduced side effect contains an antiepileptic agent selected from the group consisting of carbamazepine, gabapentin, pregabalin and valproic acid or a pharmaceutically acceptable acid addition salt or metal salt thereof in addition to BGP-15 or a pharmaceutically acceptable acid addition salt thereof and one or more conventional carrier(s).

An especially preferred pharmaceutical composition of the invention having antiepileptic activity with reduced side effect contains valproic acid or a pharmaceutically acceptable alkali metal valproate as the antiepileptic agent in addition to BGP-15 or a pharmaceutically acceptable acid addition salt thereof and one or more conventional carrier(s).

The effect of known antipsychotics as well as BGP-15 on the body weight gain was examined in the following Examples.

### Example 1

### Effect of BGP-15 on the body weight gain induced by olanzapine in rats

Groups of female Wistar rats were treated with vehicle (control group) and the compounds to be tested for 28 days. Each group consisted of 6 animals fed with normal laboratory chow and tap water *ad libitum.* The compounds to be tested were administered twice daily, at 8 h and 18 h, perorally. The antipsychotic olanzapine was administered in a dose of 1 mg/kg to induce body weight gain. BGP-15 was administered in a dose of 10 mg/kg, alone and together with olanzapine. The oral antidiabetics metformin (100 mg/kg) and rosiglitazone (3 mg/kg) were employed as reference compounds, alone and together with olanzapine. The average starting weight of the animals was 171 g. The weights of the animals at the end of the test on the 28^{th} day are listed in Table 1.

**Table 1**

| Treatment | Body weight (average in the group) in g |
|---|---|
| Control | 255 |
| Olanzapine, 1 mg/kg | 330 |
| BGP-15 dihydrochloride, 10 mg/kg | 242 |
| Metformin, 100 mg/kg | 266 |
| Rosiglitazone, 3 mg/kg | 284 |
| Olanzapine, 1 mg/kg + BGP-15 dihydrochloride, 10 mg/kg | 262 |
| Olanzapine, 1 mg/kg + metformin, 100 mg/kg | 331 |
| Olanzapine, 1 mg/kg + rosiglitazone, 3 mg/kg | 359 |

The weight gain of the control group relative to the starting weight during the test period of 28 days can be considered as normal in case of rats. The group treated with olanzapine had a significantly greater average weight than the control group. This is consistent with the obesity inducing effect of olanzapine observed in patients treated with this drug. Treatment with BGP-15 alone produced somewhat lower average weight, while treatment with metformin and rosiglitazone, respectively, produced somewhat higher average weight relative to the control group. Treatment with metformin did not reduce, while treatment with rosiglitazone increased the weight gain induced by olanzapine. However, treatment with BGP-15 dihydrochloride prevented the weight gain induced by olanzapine.

### Example 2

### Effect of BGP-15 on the body weight gain induced by olanzapine or clozapine in mice

Groups of female NMRI mice were treated with vehicle (control group) and the compounds to be tested for 15 days, perorally. Each group consisted of 10 animals fed with normal laboratory chow and tap water *ad libitum.* Treatments were performed between 5 and 6 pm, shortly before the dark phase, the primary feeding period of the day. The antipsychotic olanzapine was administered in a dose of 0.5 mg/kg, while the antipsychotic clozapine was administered in a dose of 1 mg/kg to induce body weight gain. BGP-15 was administered in a dose of 10 mg/kg, alone and together with olanzapine and clozapine, respectively. The weights of the animals were recorded twice weekly and the change in the body weights of the animals between the first and 15^{th} days are given in Table 2.

**Table 2**

| Treatment | Body weight increase (average in the group) in g |
|---|---|
| Control | 2.98 |
| Olanzapine, 0.5 mg/kg | 3.5 |
| Clozapine, 1 mg/kg | 4.11 |
| BGP-15 dihydrochloride, 10 mg/kg | 2.85 |
| Olanzapine, 0.5 mg/kg + BGP-15 dihydrochloride, 10 mg/kg | 2.33 |
| Clozapine, 1 mg/kg + BGP-15 dihydrochloride, 10 mg/kg | 2.19 |

Both antipsychotic drugs caused increased body weight gain relative to the control group. BGP-15 alone reduced body weight gain somewhat. In combination with the antipsychotic drugs, BGP-15 prevented the weight increasing side effect thereof and even further reduced the body weight change relative to the control group.

Thus, the above tests indicate that BGP-15 can effectively reduce the weight gain induced by antipsychotics, while the known oral antidiabetic drugs having also insulin sensitizing effect metfonmin and rosiglitazone used as reference agents were of no useful effect. Consequently, BGP-15 can be used to effectively prevent or reduce weight gain, overweight or obesity.

### Example 3

### Effect of BGP-15 on the body weight gain induced by risperidone in rats

The experiments were carried out in eight-week-old female Wistar rats. Each test group consisted of 10 animals fed with normal laboratory chow and tap water *ad libitum.* The animals were treated with vehicle (control group) and the compounds to be tested for 21 days. The antipsychotic risperidone was injected subcutaneously once daily in doses of 0.005 and 0.05 mg/kg, respectively to induce body weight gain. BGP-15 dihydrochloride was administered in a dose of 20 mg/kg, perorally, once daily, alone and together with risperidone.

The average starting weight of the animals was 195 g. The weights of the animals at the end of the test on the 21^{th} day are listed in Table 3

**Table 3**

| Treatment | Body weight gain in g |
|---|---|
| Control | 27 |
| BGP-15 dihydrochloride 20 mg/kg p.o. | 22.7 |
| Rispendone 0.005 mg/kg s.c. | 39.7 |
| Risperidone 0.05 mg/kg s:c. | 41 |
| Risperidone 0.005 mg/kg s.c. + BGP-15 dihydrochloride 20 mg/kg p.o. | 25.8 |
| Risperidone 0.05 mg/kg s.c. + BGP-15 dihydrochloride 20 mg/kg p.o. | 28.7 |

Both doses of the antipsychotic drug risperidone caused increased body weight gain relative to the control group. BGP-15 alone reduced body weight gain somewhat. In combination with the antipsychotic drug, BGP-15 prevented the weight increasing side effect thereof in both doses.

## Claims

1. Use of O-(3-piperidino-2-hydroxy-1-propyl)-nicotinic amidoxime or a pharmaceutically acceptable acid addition salt thereof for the preparation of a pharmaceutical composition for preventing or reducing the side effect leading to overweight or obesity in medication with a known antipsychotic, antidepressant or antiepileptic drug.

2. A use of Claim 1 in which O-(3-piperidino-2-hydroxy-1-propyl)-nicotinic amidoxime dihydrochloride is to be administered.

3. A use of Claim 1 in which the antipsychotic drug is selected from olanzapine, clozapine, risperidone, quetiapine and sulpiride or a pharmaceutically acceptable acid addition salt thereof.

4. A pharmaceutical composition having antipsychotic, antidepressant or antiepileptic activity with reduced side effect comprising a known antipsychotic or antidepressant or antiepileptic agent and O-(3-piperidino-2-hydroxy-1-propyl)-nicotinic amidoxime or a pharmaceutically acceptable acid addition salt thereof in admixture with one or more conventional carrier(s).

5. A pharmaceutical composition of Claim 4 in which the antipsychotic agent is selected from olanzapine, clozapine, risperidone, quetiapine and sulpiride or a pharmaceutically acceptable acid addition salt thereof.

6. A pharmaceutical composition of Claim 4 or 5 comprising olanzapine and O-(3-piperidino-2-hydroxy-1-propyl)-nicotinic amidoxime or a pharmaceutically acceptable acid addition salt thereof.

7. A pharmaceutical composition of Claim 4 or 5 comprising clozapine and O-(3-piperidino-2-hydroxy-1-propyl)-nicotinic amidoxime or a pharmaceutically acceptable acid addition salt thereof.

8. A pharmaceutical composition of Claim 4 or 5 comprising risperidone and O-(3-piperidino-2-hydroxy-1-propyl)-nicotinic amidoxime or a pharmaceutically acceptable acid addition salt thereof.

9. A pharmaceutical composition of Claim 4 in which the antidepressant agent is selected from clomipramine, citalopram, fluoxetine, fluvoxamine, paroxetine and sertraline or a pharmaceutically acceptable acid addition salt thereof.

10. A pharmaceutical composition of Claim 4 in which the antiepileptic agent is valproic acid or a pharmaceutically acceptable alkali salt thereof.

## Patentansprüche

1. Verwendung von O-(3-Piperidino-2-hydroxy-1-propyl)-nicotinsäure-amidoxim oder einem pharmazeutisch zulässigen Säureadditionssalz davon für die Herstellung einer pharmazeutischen Zusammensetzung zur Verhinderung oder Verringerung der Nebenwirkungen, die zu Übergewicht oder Fettleibigkeit bei der Medikation mit einem bekannten antipsychotischen, antidepressiven oder antiepileptischen Arzneistoff führen.

2. Verwendung gemäß Anspruch 1, bei der O-(3-Piperidino-2-hydroxy-1-propyl)-nicotinsäure-amidoxim-dihydrochlorid verabreicht werden soll.

3. Verwendung gemäß Anspruch 1, wobei der antipsychotische Arzneistoff aus Olanzapin, Clozapin, Risperidon, Quetiapin und Sulpirid oder einem pharmazeutisch zulässigen Säureadditionssalz davon gewählt ist.

4. Pharmazeutische Zusammensetzung mit antipsychotischer, antidepressiver oder antiepileptischer Aktivität mit reduzierter Nebenwirkung, umfassend ein bekanntes antipsychotisches oder antidepressives oder antiepileptisches Mittel und O-(3-Piperidino-2-hydroxy-1-propyl)-nicotinsäure-amidoxim oder ein pharmazeutisch zulässiges Säureadditionssalz davon in Vermischung mit einem oder mehreren herkömmlichen Träger(n).

5. Pharmazeutische Zusammensetzung gemäß Anspruch 4, in welcher das antipsychotische Mittel aus Olanzapin, Clozapin, Risperidon, Quetiapin und Sulpirid oder einem pharmazeutisch zulässigen Säureadditionssalz davon gewählt ist.

6. Pharmazeutische Zusammensetzung gemäß Anspruch 4 oder 5, welche Olanzapin und O-(3-Piperidino-2-hydroxy-1-propyl)-nicotinsäure-amidoxim oder ein pharmazeutisch zulässiges Säureadditionssalz davon umfasst.

7. Pharmazeutische Zusammensetzung gemäß Anspruch 4 oder 5, welche Clozapin und O-(3-Piperidino-2-hydroxy-1-propyl)-nicotinsäure-amidoxim oder ein pharmazeutisch zulässiges Säureadditionssalz davon umfasst.

8. Pharmazeutische Zusammensetzung gemäß Anspruch 4 oder 5, welche Risperidon und O-(3-Piperidino-2-hydroxy-1-propyl)-nicotinsäure-amidoxim oder ein pharmazeutisch zulässiges Säureadditionssalz davon umfasst.

9. Pharmazeutische Zusammensetzung gemäß Anspruch 4, in welcher das antidepressive Mittel aus Clomipramin, Citalopram, Fluoxetin, Fluvoxamin, Paroxetin und Sertralin oder einem pharmazeutisch zulässigen Säureadditionssalz davon gewählt ist.

10. Pharmazeutische Zusammensetzung gemäß Anspruch 4, in welcher das antiepileptische Mittel Valproinsäure oder ein pharmazeutisch zulässiges Alkalisalz davon ist.

## Revendications

1. Utilisation d'amidoxime O-(3-pipéridino-2-hydroxy-1-propyl)-nicotinique ou d'un sel d'addition avec un acide pharmaceutiquement acceptable de celle-ci, pour la préparation d'une composition pharmaceutique pour prévenir ou réduire l'effet secondaire conduisant à une surchage pondérale ou une obésité dans une médication avec une substance médicamenteuse antipsychotique, antidépressive ou antiépileptique connue.

2. Utilisation selon la revendication 1, dans laquelle du dichlorhydrate d'amidoxime O-(3-pipéridino-2-hydroxy-1-propyl)-nicotinique doit être administré.

3. Utilisation selon la revendication 1, dans laquelle la substance médicamenteuse antipsychotique est choisie parmi l'olanzapine, la clozapine, la rispéridone, la quétiapine et le sulpiride ou un sel d'addition avec un acide pharmaceutiquement acceptable de celle-ci.

4. Composition pharmaceutique ayant une activité antipsychotique, antidépressive ou antiépileptique ayant un effet secondaire réduit, comprenant un agent antipsychotique ou antidépresseur ou antiépileptique connu et de l'amidoxime O-(3-pipéridino-2-hydroxy-1-propyl)-nicotinique ou un sel d'addition avec un acide pharmaceutiquement acceptable de celle-ci en mélange avec un ou plusieurs support(s) traditionnel(s).

5. Composition pharmaceutique selon la revendication 4, dans laquelle l'agent antipsychotique est choisi parmi l'olanzapine, la clozapine, la rispéridone, la quétiapine et le sulpiride ou un sel d'addition avec un acide pharmaceutiquement acceptable de celui-ci.

6. Composition pharmaceutique selon la revendication 4 ou 5, comprenant de l'olanzapine et de l'amidoxime O-(3-pipéridino-2-hydroxy-1-propyl)-nicotinique ou un sel d'addition avec un acide pharmaceutiquement acceptable de ceux-ci.

7. Composition pharmaceutique selon la revendication 4 ou 5, comprenant de la clozapine et de l'amidoxime O-(3-pipéridino-2-hydroxy-1-propyl)-nicotinique ou un sel d'addition avec un acide pharmaceutiquement acceptable de ceux-ci.

8. Composition pharmaceutique selon la revendication 4 ou 5, comprenant de la rispéridone et de l'amidoxime O-(3-pipéridino-2-hydroxy-1-propyl)-nicotinique ou un sel d'addition avec un acide pharmaceutiquement acceptable de ceux-ci.

9. Composition pharmaceutique selon la revendication 4, dans laquelle l'agent antidépresseur est choisi parmi la clomipramine, le citalopram, la fluoxétine, la fluvoxamine, la paroxétine et la sertraline ou un sel d'addition avec un acide pharmaceutiquement acceptable de celui-ci.

10. Composition pharmaceutique selon la revendication 4, dans laquelle l'agent antiépileptique est l'acide valproïque ou un sel alcalin pharmaceutiquement acceptable de celui-ci.
